# EUROPEAN PATENT APPLICATION

(11) **EP 2 186 484 A1**
(43) Date of publication of application: **19.05.2010**
(21) Application number: 08075877.4
(22) Date of filing: 13.11.2008
(51) Int. Cl.: A61B 10/00

(54) **Container for transporting test tubes**

(71) Applicant: Necontra B.V., 7576 EE Oldenzaal (NL)
(72) Inventor: Posthumus, WillemPetrus Albertus, 7576 EE Oldenzaal (NL)
(74) Representative: 't Jong, Bastiaan Jacob

(57) **Abstract**

The invention relates to a container (1) for transporting test tubes, the container comprising:
- a rigid shell having a base shell portion (2) and a lid shell portion (3) hingedly arranged to the base shell portion;
- a thermally insulating lining (7) arranged on the inner wall of the base portion and a thermally insulating lining (8) arranged on the lid portion;
- a seal arranged on either thermally insulating lining, such that in closed position of the container, the seal is compressed between the thermally insulating linings, providing a fluid tight shell.

## Description

The invention relates to a complete transport container for transporting test tubes or primary biological material from humans or animals.

Such complete transport containers are well known in the field and comply with strict regulations (ADR 2007). These strict regulations were that containers with primary biological material from humans or animals should be transported using a primary container or test tube, a secondary leakage free packaging with sufficient absorbing material to absorb the complete content of all primary containers and a tertiary rigid outer or transport container. The complete transport container should withstand specific tests: drop tests from a height of at least 1,2m and a pressure difference of 95kPa(see ADR 2007). After applying the specific tests it is not allowed that any fluid is leaking out of the tertiary transport container.

In practice, the now used leakage free bags provide in daily use a large problem when the bag had to be opened frequently. For example with a person traveling to patients at home, the person had to reopen the bag at each home to add a test tube to the container. According to the regulations, the person should renew the bag at each reopening of the bag, but it was also allowed to use a clip to seal and reopen the bag.

Recently the regulations were changed in that the requirement for a bag was removed.As from 1 january 2007 the ADR (Economic Commision for Europe - Working Party on the Transport of Dangerous Goods, ECE/Trans/185 inclusive Annexes A and B) describes a secondary leakage free packaging compared to the older description of a leakage free secondary bag. However it is still required that the container is fluid tight, such that when a test tube breaks, the content can not leak out of the container.

Accordingly it is the object of the invention to provide a container for transporting test tubes, which complies with the regulations and which reduces the disadvantages of the containers of the prior art.

This object is achieved with a container according to the invention, the container comprising:
- a rigid shell having a base shell portion and a lid shell portion hingedly arranged to the base shell portion;
- a thermally isolating lining arranged on the inner wall of the base portion and a thermally isolating lining arranged on the lid portion;
- a sealing arranged on either thermally isolating lining, such that in closed position of the container, the sealing is compressed between the thermally isolating linings, providing a fluid tight shell.

By providing a sealing which is compressed between the thermally isolating linings when the container is in the closed position, the cavity enclose by the linings and the shell is fluid tight. This has the additional advantage that also no heat leaks are present, as the cavity is fully enclosed by the thermally isolating lining.

In an embodiment of the container of the invention, the thermally isolating linings are suitably shaped blocks of isolating material. Preferably, the isolating material is expanded polystyrene (EPS).

EPS can be manufactured very accurately, such that it has a tight fit in the rigid shell and that compression of the sealing can be ensured.

In a preferred embodiment of the container according to the invention, the isolating linings both have a corresponding contact surface, with which both linings abut in the closed position of the container.

Preferably, at least one of the contact surfaces is provided with a groove, in which a sealing ring is arranged. This sealing ring can be arranged in the groove by a tight fit or by an adhesive.

In yet another preferred embodiment of the container according to the invention the effective height of both the isolating linings and the sealing is larger than the effective inner height of the rigid shell. This ensures that when the lid shell portion is closed, the isolating linings and the sealing must be compressed to close the container. As a result, the sealing provides a fluid tight sealing.

The invention also relates to a combination of a container according to the invention and a tray for test tubes, said tray being removably arranged in the shell of the container. With the tray the test tubes can be removed easily from and inserted into the container.

In a preferred embodiment of the combination according to the invention, the tray for test tubes comprises a support plate having at least two upstanding walls and at least one test tube rack arranged on the support plate. By arranging test tube racks on a support plate, it is possible to remove all the racks at once from the container.

Preferably the combination according to the invention further comprises a handle, which is connectable to the two upstanding walls of the support plate to remove the tray from the container.

In yet another preferred embodiment of the combination according to the invention the two upstanding walls are provided with a folded back edge and the walls are resilient such that the two walls bend towards each other when the handle is pressed onto the two walls and the handle catches onto the folded back edges.

By pressing the handle onto the upstanding walls a quick connection of the handle with the support plate is achieved. As the walls are resilient it is also ensured that the connection remains when the support plate is lifted out of the container. Only by sliding the handle sideways the connection between the handle and the upstanding walls can be removed.

In yet another embodiment of the combination according to the invention the bottom of the container is provided with a cavity for storage of ice packs.

These and other advantages of the invention will be elucidated in conjunction with the accompanying drawings.
Figure 1 shows in perspective view a container according to the invention.
Figures 2A and 2B show in perspective view the bottom thermal lining of the container according to figure 1.
Figures 3A and 3B show in perspective view the upper thermal lining of the container according to figure 1.
Figure 4 shows a side view of the linings.
Figure 5 shows a cross sectional view along the line V-V shown in figure 4.
Figure 6 shows another side view of the linings.
Figure 7 shows a cross sectional view along the line VII-VII shown in figure 6.
Figure 8 shows the content of the container of a combination according to the invention.

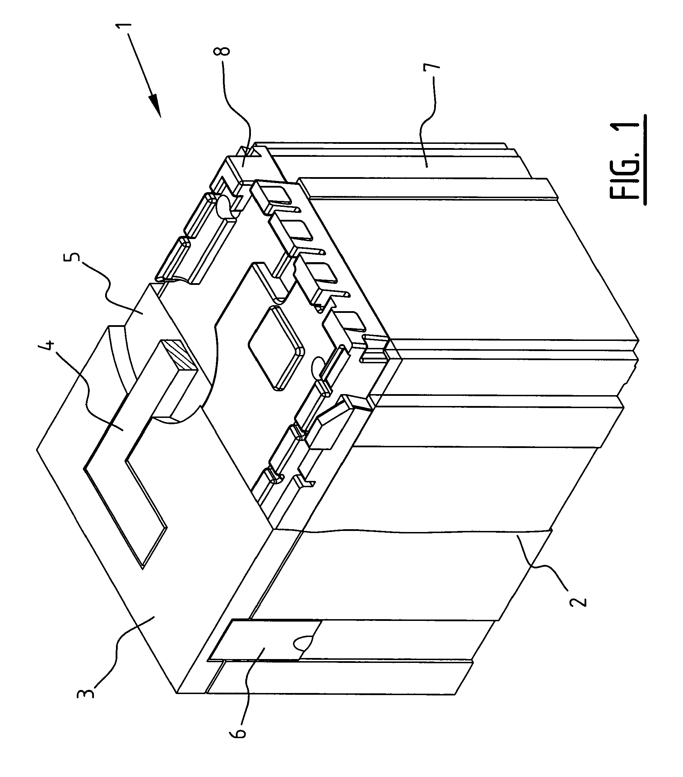

In figure 1 a container 1 according to the invention is shown in perspective view. This container 1 has a base shell portion 2 and a lid shell portion 3. The lid shell portion 3 is provided with a handle 4, which is integrated in to the upper surface of the lid shell portion 3. In order to be able to grab the handle 4 a recess 5 is provided.

The lid shell portion 3 and base shell portion 2 are hingedly connected to each other and a latch 6 is provided to lock the lid shell portion 3 to the base shell portion 2.

In the base shell portion 2 a thermally isolating liner 7 is arranged. Also in the lid shell portion a thermally isolating liner 8 is arranged. The thermally isolating liners 7 and 8 are preferably shaped such that the contours of the inner walls of the base shell portion 2 and lid shell portion 3 respectively are followed. These liners 7 and 8 are made of expanded polystyrene (EPS).

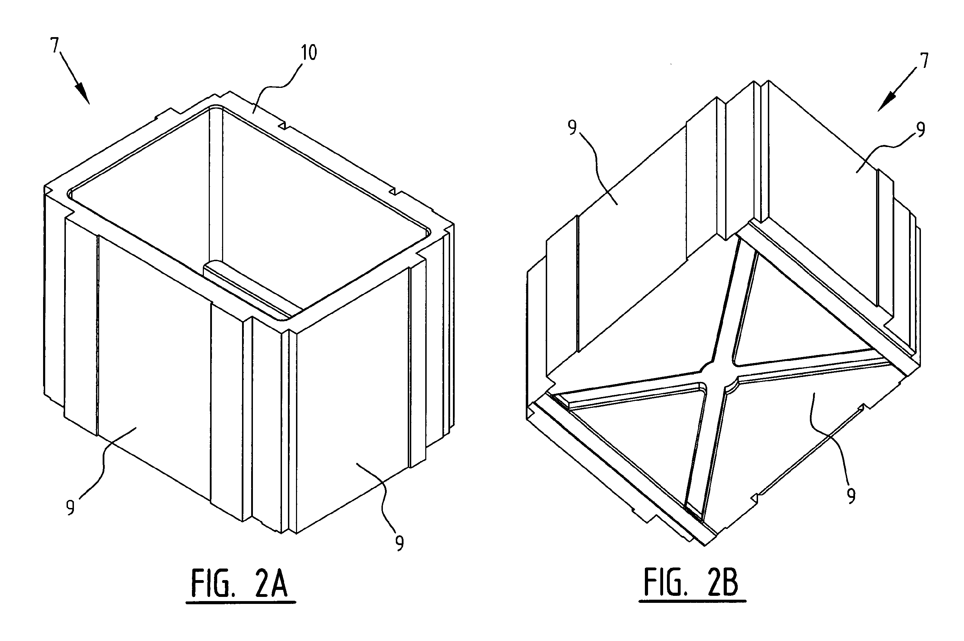

In figures 2A and 2B the thermally isolating liner for the base shell portion 2 is shown. The outer walls 9 are provided with recesses and ridges to follow the internal surfaces of the base shell portion 2. The liner 7 is furthermore provided with a contact surface 10. This contact surface 10 contacts the lid liner 8 and contributes to a fluid tight sealing of the container 1.

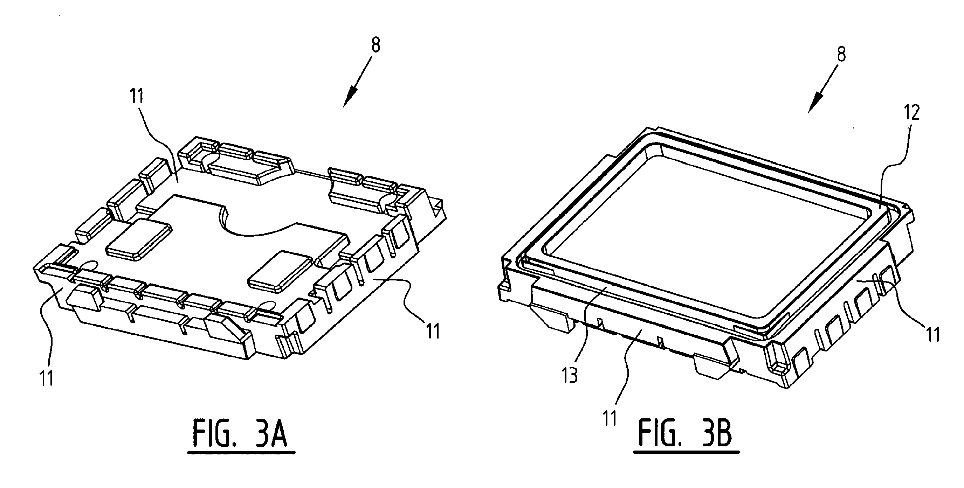

In figures 3A and 3B the thermally isolating liner 8 for the lid shell portion 3 is shown. The outer walls 11 of the liner 8 are also provided with recesses and ridges to follow the internal surfaces of the lid shell portion 3. The liner 8 is also provided with a contact surface 12 which contacts the contact surface 10 in the closed position of the container 1. The contact surface 12 is provided with a circumferential groove 13, in which a sealing can be arranged.

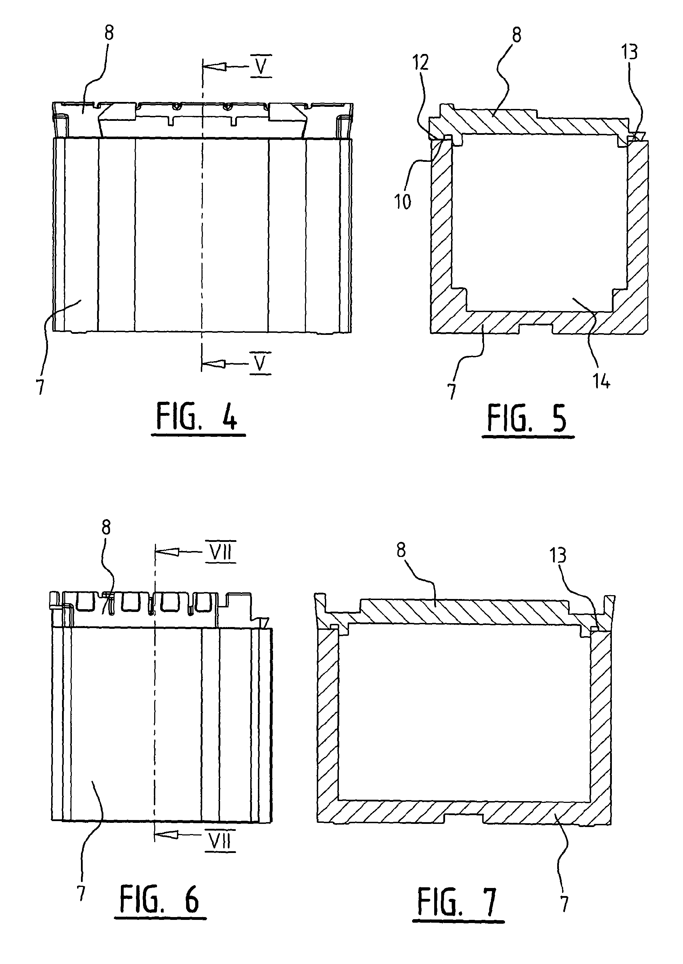

In the figures 4, 5, 6 and 7 side views and cross sectional views of the liners 7 and 8 are shown. From these figures it is clear that the contact surfaces 10 and 12 of the liners 7 and 8 respectively abut when the container is in the closed position. The sealing (not shown) provided in the groove 13 is compressed between the liners 10 and 12, such that a fluid tight sealing is achieved.

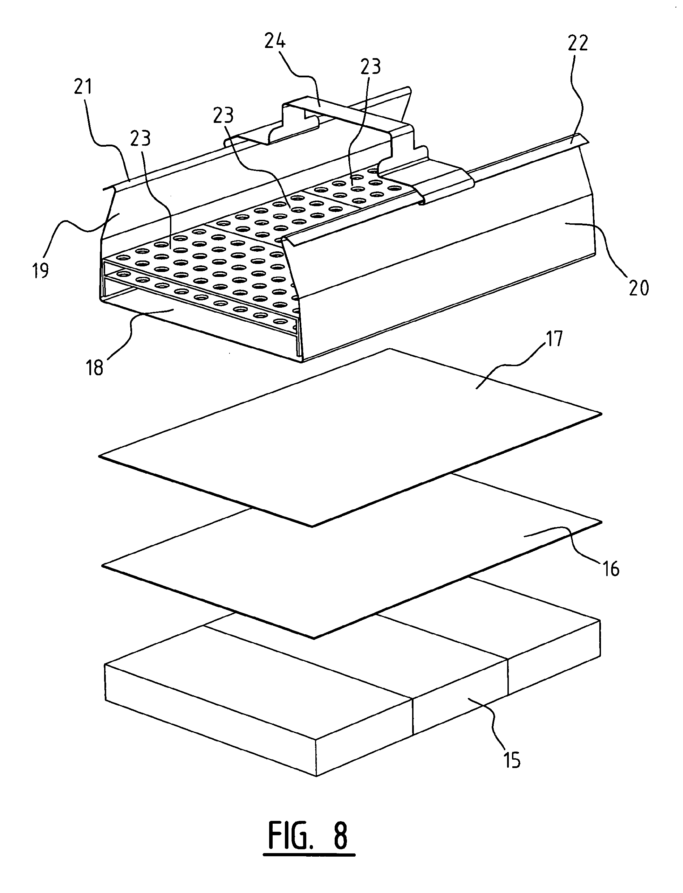

In figure 8 the content for a combination according to the invention is shown. An ice pack 15 is placed in the recess 14 in the bottom of the liner 7 (see figure 5). Over this ice pack 15 a plastic sheet 16 is placed, which prevents direct contact between the other parts within the container.

Furthermore an absorption sheet 17 is placed on top of the plastic sheet 16. This absorption sheet 17 is required according to the regulations and absorbs liquid when some is spilled or a test tube breaks.

On top of the absorption sheet 17 a support plate 18 is arranged. This support plate has two upstanding walls 19 and 20, which are each provided with folded back edges 21 and 22 respectively. On the support plate 18 test tube racks 23 are placed.

The support plate 18 together with the test tube racks 23 can be lifted out of the container by pressing the handle 24 onto the edges 21 and 22, such that the handle 24 catches on the edges 21 and 22. Due to the resiliency of the upstanding walls 19 and 20, the connection with the handle 24 is maintained. The handle 24 can be removed from the support plate 18, by sliding the handle sideways.

## Claims

1. Container for transporting test tubes, the container comprising:
- a rigid shell having a base shell portion and a lid shell portion hingedly arranged to the base shell portion;
- a thermally isolating lining arranged on the inner wall of the base portion and a thermally isolating lining arranged on the lid portion;
- a sealing arranged on either thermally isolating lining, such that in closed position of the container, the sealing is compressed between the thermally isolating linings, providing a fluid tight shell.

2. Container according to claim 1, wherein the thermally isolating linings are suitably shaped blocks of isolating material.

3. Container according to claim 2, wherein the isolating material is expanded polystyrene (EPS).

4. Container according to any of the preceding claims, wherein the isolating linings both have a corresponding contact surface, with which both linings abut in the closed position of the container.

5. Container according to claim 4, wherein at least one of the contact surfaces is provided with a groove, in which a sealing ring is arranged.

6. Container according to any of the preceding claims, wherein the effective height of both the isolating linings and the sealing is larger than the effective inner height of the rigid shell.

7. Combination of a container according to any of the preceding claims and a tray for test tubes, said tray being removably arranged in the shell of the container.

8. Combination according to claim 7, wherein the tray for test tubes comprises a support plate having at least two upstanding walls and at least one test tube rack arranged on the support plate.

9. Combination according to claim 8, further comprising a handle, which is connectable to the two upstanding walls of the support plate to remove the tray from the container.

10. Combination according to claim 9, wherein the two upstanding walls are provided with a folded back edge and wherein the walls are resilient such that the two walls bend towards each other when the handle is pressed onto the two walls and the handle catches onto the folded back edges.

11. Combination according to any of the preceding claims 7 - 10, wherein the bottom of the container is provided with a cavity for storage of ice packs.
